# EUROPÄISCHE PATENTSCHRIFT

(11) **EP 1 796 567 B1**
(45) Veröffentlichungstag und Bekanntmachung des Hinweises auf die Patenterteilung: **18.04.2012**
(21) Anmeldenummer: 05769843.3
(22) Anmeldetag: 07.07.2005
(51) Int. Cl.: A61B 18/14

(54) **BIPOLARE KOAGULATIONSELEKTRODE**
BIPOLAR COAGULATION ELECTRODE
ELECTRODE DE COAGULATION BIPOLAIRE

(30) Priorität: 07.07.2004 DE 102004033595
(43) Veröffentlichungstag der Anmeldung: 20.06.2007
(73) Patentinhaber: Celon AG Medical Instruments, 14513 Teltow (DE)
(72) Erfinder: DESINGER, Kai, 12157 Berlin (DE); STEIN, Thomas, 10787 Berlin (DE); ROGGAN, André, 12163 Berlin (DE)
(74) Vertreter: Eisenführ, Speiser & Partner
(86) Internationale Anmeldenummer: PCT/EP2005/053255
(87) Internationale Veröffentlichungsnummer: WO 2006/003216

(56) Entgegenhaltungen:
- US-A- 4 326 529
- US-A- 4 381 007
- US-A- 5 217 459
- US-A1- 2003 120 269
- US-A1- 2004 116 793
- US-B1- 6 413 255

## Beschreibung

Die Erfindung betrifft eine bipolare Koagulationssonde, die sich für den Einsatz in minimal-invasiver Chirurgie eignet.

Die Versorgung peri-operativer Blutungen ist Teil eines jeden chirurgischen Eingriffs. Die bipolare Hochfrequenzkoagulation hat sich in den letzten Jahren zu einer Standard-Methode zur Blutstillung entwickelt. Die eingesetzten Koagulationselektroden umfassen mindestens zwei Elektrodenpole, zwischen denen eine hochfrequente Wechselspannung angelegt werden kann und die in einem gemeinsamen Instrument untergebracht sind. Die beiden Elektrodenpole liegen beispielsweise auf gegeneinander isolierten Branchen einer bipolaren Pinzette oder können in einer koaxialen Anordnung in einem sogenannten Koagulations-Stift verwirklicht werden. Ein Vorteil der bipolaren Technik besteht darin, dass ein Stromfluss nur zwischen den dicht benachbarten Polen der Elektroden erfolgt und nicht durch den gesamten Körper des Patienten. Auch sind Verbrennungen durch unsachgemäße befestigte Neutralelektroden oder unbeabsichtigte Erdung des Patienten, wie sie in der monopolaren Hochfrequenzchirurgie möglich sind, ausgeschlossen.

Für einige Gebiete der minimal-invasiven Chirurgie, insbesondere der endoskopischen Neurochirurgie, eignen sich die bisherigen bipolaren Koagulationselektroden mit herkömmlicher Formgebung nicht. Es sind daher bipolare Koagulationssonden mit einer sphärischen Stirnfläche entwickelt worden, wobei in die Stirnfläche ein Pol einer ersten Elektrode und ein Pol einer zweiten Elektrode integriert ist. Mit der sphärischen Formgebung der Stirnfläche wird der Einsatz der Koagulationssonde in der minimal-invasiven Chirurgie wesentlich erleichtert. Die kugelförmige Oberfläche der Stirnfläche ermöglicht eine besonders einfache Handhabung der Koagulationssonde. Die sphärische Stirnfläche ermöglicht weiterhin auch dann eine Oberflächenkoagulation, wenn der Schaft nicht senkrecht zur Gewebeoberfläche gehalten wird.

Die bekannten bipolaren Koagulationssonden mit einer aktiven Stirnfläche weisen jedoch den Nachteil auf, dass das Gewebe beim bestimmungsgemäßen Gebrauch an den Polen der Elektroden anhaften kann.

In der US-amerikanischen Patentschrift US 4,381,007 ist eine multipolare elektronische Sonde beschrieben. Die Sonde soll zur Verformung einer Komea eines menschlichen Auges dienen. Die Sonde umfasst eine Kühlvorrichtung, die ausgebildet ist, ein Kühlmittel durch eine zentral liegende Elektrode der Sonde auf die Oberfläche der Komea aufzubringen. Die Sonde ist an ihrem distalen Ende derart geformt, dass sie bei Kontakt mit dem Auge eines Patienten in etwa formschlüssig auf dem Auge aufliegt. Die in US 4,381,007 beschriebene Sonde weist die Merkmale des Oberbegriffs des Anspruchs 1 auf.

In der US-amerikanischen Patentschrift US 6,4123,255 B1 ist eine Vorrichtung zur Durchführung dermatologischer Behandlungen beschrieben. Insbesondere eignet sich die Vorrichtung zur Verformung und Neugestaltung von Weichgewebe. Die Vorrichtung umfasst eine mono- oder bipolare Elektrode, die in Form einer Hülse ausgestaltet sein kann. Es ist ferner offenbart, dass über eine zum Patienten zeigende Außenfläche der Elektrode elektrische Energie kontrolliert auf das zu behandelnde Gewebe übertragen wird. Zur Vermeidung von Verbrennungen am Weichgewebe sieht die Vorrichtung außerdem eine Kühleinrichtung vor, die ausgebildet ist, die Innenfläche der Elektrode mit einer Kühlflüssigkeit zur besprühen.

US2004/0116793 A1 offenbart eine Ablationssonde mit zwei konzentrisch angeordneten Ringelektroden an der Sondenspitze.

Der vorliegenden Erfindung liegt die Aufgabe zugrunde, eine Alternative zu den bekannten Koagulationssonden zu schaffen, die Stabil ist und geringe Herstellungskoffen verursacht.

Erfindungsgemäß wird diese Aufgabe durch eine bipolare Koagulationssonde nach Anspruch 1 gelöst. Die erfindungsgemäße bipolare Koagulationssonde besitzt einen Schaft mit einem distalen Ende, welches eine Stirnfläche aufweist, in die ein Pol einer ersten Elektrode und ein Pol einer zweiten Elektrode integriert sind. Die bipolare Koagulationselektrode zeichnet sich dadurch aus, dass sie eine Temperiereinheit umfasst. Auf diese Weise kann das aus der konventionellen Elektrochirurgie bekannte nachteilige Anhaften des Gewebes an den Polen der Elektroden gemindert oder gar vollständig vermieden werden. Bei einer nicht temperierten Koagulationssonde herrscht während der Koagulation die höchste Temperatur in dem Gewebsbereich, der an den Polen der Elektroden anliegt. Mit zunehmender Austrocknung beginnt das Gewebe an den Polen der Elektroden anzuhaften. Mittels der erfindungsgemäßen Temperiereinheit kann jedoch die Temperatur an den Polen soweit reduziert werden, dass ein Ankleben des Gewebes nicht mehr möglich ist. Durch die Temperierung der Pole der Elektroden auf eine Temperatur, die unter der für die Gewebeanhaftung kritischen Temperatur liegt, wird die Zone der kritischen Temperaturen von den Polen weg in tiefere Gewebsschichten verschoben. In diesem Fall dient die Temperiereinheit dem Kühlen wenigstens des distalen Endes der Koagulationssonde. Ferner lassen sich mit der erfindungsgemäßen Temperiereinheit die Temperaturen der Stirnfläche der Elektrodenspitze während und vor der Koagulation sowie bei der Einführung der Elektrode in den Körper einstellen und aufrechterhalten. Insbesondere ist es auch möglich, die Koagulationssonde vor einer Applikation zu erwärmen. Eine Koagulationssonde ohne Temperiereinheit erwärmt sich erst bei Gewebekontakt, da erst dann ein Hochfrequenzstrom fließen kann, der zu einer Gewebeerwärmung und damit indirekt zu einer Erwärmung der Koagulationssonde führt. Für einige Applikationen ist es jedoch wünschenswert, wenn die Koagulationssonde bereits unmittelbar zu Beginn einer Applikation eine gewünschte Temperatur aufweist und nicht erst nach einer durch die indirekte Erwärmung bedingten Verzögerung.

Der elektrische Pol der zweiten Elektrode ist ringförmig ausgebildet. Der Pol der ersten Elektrode ist ferner innerhalb des ringförmigen Pols der zweiten Elektrode angeordnet. Durch die Formgebung und relative Anordnung der beiden Pole auf der Stirnfläche lassen sich örtlich klar umgrenzte Bereiche des anliegenden Gewebes mit einer hochfrequenten Wechselspannungen belegen und zwar sehr gleichmäßig über die gesamte Elektrodenspitze hinweg.

Die erste Elektrode nimmt zumindest im Bereich des distalen Endes die Form einer Hülse ein, wobei das geschlossene distale Ende der ersten Elektrode den Boden der Hülse bzw. den Pol in der Stirnfläche ausbildet. Die zweite Elektrode ist im Bereich des distalen Endes rohrförmig, wobei ein distales Ende der rohrförmigen zweiten Elektrode den Pol in der Stirnfläche ausbildet. Durch die genannte Formgebung der Elektroden lässt sich die Fertigung der Koagulationselektrode vereinfachen, so dass die Herstellungskosten sinken. Zusätzlich verleiht die Formgebung der Elektrodenspitze die für die Handhabung notwendige Stabilität. Schließlich lässt sich diese Form mit einem äußeren, ringförmigen Pol mit schon rohrförmigen Elektroden besonders einfach realisieren. Die Form mit einer hülsenförmigen ersten Elektrode in Kombination mit der Temperiereinheit ist herstellungstechnisch besonders zu realisieren.

Die erfindungsgemäße Temperiereinheit ist derart ausgebildet, dass sie ein Temperiermedium zum distalen Ende der bipolaren Koagulationssonde führen kann, um die Stirnfläche zu kühlen bzw. zu heizen. Das Temperiermedium ist ein zur Aufnahme und Abgabe thermischer Energie geeignetes Fluid. Die Temperiereinheit kann übliche Mittel zum Einstellen einer vorzugebenden Temperatur oder eines vorgebbaren Temperaturprofils des Temperiermediums, wie z.B. einen ggf. steuerbaren Thermostaten umfassen oder mit solchen Mitteln verbunden sein. Für die erfindungsgemäßen Zwecke kann ein nur bereichsweises Temperieren der Stirnfläche ausreichend sein, so dass das Temperiermedium nicht zwangsläufig in thermischen Austausch mit der gesamten Stirnfläche der bipolaren Koagulationselektrode stehen muss. Im Vordergrund steht vor allem das Temperieren der in der Stirnfläche angeordneten Pole der bipolaren Koagulationselektrode.

Vorzugsweise umfasst die Temperiereinheit eine Leitstruktur für das Temperiermedium, mit der das Temperiermedium an die Rückseite der Stirnfläche im inneren des distalen Endes der Koagulationssonde geführt und dort zumindest teilweise umlenkt und zurückgeführt wird. Das Temperiermedium strömt somit an der Rückseite der Stirnfläche entlang und führt auf diese Weise die bei der Koagulation entstehende Wärme rasch und effektiv ab oder heizt die Stirnfläche auf die gewünschte Temperatur.

Das Temperiermedium für die Temperiereinheit ist nach einer bevorzugten ersten Variante eine sterile, pyrogenfreie, physiologische Kochsalzlösung. Hierdurch wird verhindert, dass bei einem Defekt der Koagulationselektrode und dem damit verbundenen Austreten des Temperiermediums in den Patienten eine gesundheitliche Gefährdung besteht. Weiterhin ist das Temperiermedium elektrisch leitfähig. Sind daher nach einer weiterführenden bevorzugten Ausführungsform der Erfindung an der Stirnfläche der Elektrodenspitze Bohrungen, insbesondere in den Polen, vorhanden, durch die das Temperiermedium zumindest teilweise austritt, so kann die bei starker Erwärmung des Gewebes einsetzende Gewebsaustrocknung kompensiert werden und der elektrische Energieeintrag durch Aufrechterhaltung der elektrischen Leitfähigkeit des Gewebes verbessert werden.

Nach einer bevorzugten zweiten Variante wird als Temperiermedium deionisiertes Wasser eingesetzt. Dies hat den Vorteil, dass auf eine elektrische Isolierung der Pole gegenüber dem Temperiermedium verzichtet werden kann, denn die Leitfähigkeit von deionisiertem Wasser ist für die erfindungsgemäßen Zwecke hinreichend niedrig. Ein Kühlen und Beheizen der Stirnfläche, insbesondere unmittelbar im Bereich der Pole, kann auf diese Weise sehr effektiv und ohne aufwendige bauliche Anpassung des Elektrodenaufbaus realisiert werden.

Vorzugsweise ist die Temperiereinheit mit einer Schlauchpumpe zum Fördern des Temperiermediums verbunden. Schlauchpumpen haben den Vorteil, dass die Mechanik der Pumpe nicht mit der zu pumpenden Flüssigkeit in Berührung kommt. Sterile Flüssigkeiten bleiben somit steril, vorausgesetzt es wird ein steriler Schlauch verwendet. Das Aufrechterhalten der Sterilität des Temperiermediums erhöht die Sicherheit des Systems. Bei fehlerhaftem Austreten des Temperiermediums in den Patienten besteht somit kein Infektionsrisiko.

Die Stirnfläche ist die von einem Blickpunkt auf der in distaler Richtung verlängerten Längsachse des Schaftes aus sichtbare Fläche, wenn der Blick in proximale Richtung des Schaftes gelenkt ist.

Nach einer weiteren bevorzugten Ausführungsform der Erfindung ist die Stirnfläche der erfindungsgemäßen Koagulationssonde sphärisch geformt. Unter "sphärischer Stirnfläche" wird ein zumindest annähernd kugelförmiger Bereich der Elektrodenspitze verstanden. Dabei kann die sphärische Stirnfläche zum Beispiel als Halbkugel das distale Ende des Schaftes bilden. Die Formgebung ist jedoch nicht auf im geometrischen Sinne kugelförmige Konturen beschränkt, sondern kann auch eine davon abweichende Krümmung der Oberfläche besitzen. Die sphärische Stirnfläche ist in Richtung des sich anschließenden Schaftes der Elektrodenspitze abgerundet und weist keine senkrecht zur Längsachse verlaufenden Abschnitte auf. Die Oberfläche der sphärischen Stirnfläche muss nicht völlig glatt sein, sondern kann auch in geringem Maße konturiert sein, beispielsweise durch geringes Anheben der Pole der Elektroden aus der Ebene der Stirnfläche.

Als "Pol" wird der Teil der Elektrode bezeichnet, der in der Applikation eine elektrisch aktive Oberfläche zum umgebenden Gewebe bildet, d.h. derjenige Oberflächenbereich, in dem es bei Anliegen einer Wechselspannung zum Übertritt elektrischer Energie in das umgebende Gewebe kommt.

Eine weitere - auch ohne die erfindungsgemäße Temperiereinheit ausführbare - bevorzugte Ausführungsform der bipolaren Koagulationssonde besitzt ebenfalls ein distales Endes, das eine Stirnfläche aufweist, in die ein Pol einer ersten Elektrode und ein Pol einer zweiten Elektrode integriert sind. Die bipolare Koagulationssonde ist daher zur Oberflächenkoagulation geeignet. Zudem umfasst die bipolare Koagulationselektrode zumindest eine weitere Elektrode mit einem am Umfang des Schaftes angeordneten Pol. Ein zweiter Pol auf dem Umfang des Schaftes kann von dem in proximale Richtung verlängerten, ringförmigen Pol in der Stirnfläche der Koagulationssonde gebildet sein, d.h. der sich an die Stirnfläche anschließende Schaft der Koagulationssonde weist mindestens zwei Pole auf. Die Koagulationssonde besitzt somit zwei Elektroden, die zur interstitiellen Koagulation geeignet sind. Vorzugsweise läuft ein am Umfang der Elektrodenspitze angeordneter Pol ringförmig um den Schaft, da hierdurch eine räumlich klar definierte Behandlung des Gewebes ermöglicht wird. Nach einer schaltungstechnisch besonders einfach zu realisierenden, sowohl zur interstitiellen als auch bipolaren Oberflächenkoagulation geeigneten Variante bildet eine der Elektroden der Koagulationselektrode sowohl einen Pol in der Stirnfläche als auch einen Pol am Umfang der Elektrodenspitze. Auf diese Weise entsteht de facto ein gemeinsamer Pol mit zwei Polflächen, so dass eine kombinierte Koagulationssonde für die bipolare Oberflächen- und die bipolare interstitielle Koagulation insgesamt drei Pole aufweist, von denen zwei nicht benachbarte Pole elektrisch miteinander verbunden sein können. Die Koagulationssonde besitzt in dieser Ausführungsform vorzugsweise nur drei Elektroden. Die vorgenannte besondere Ausführungsform der bipolaren Koagulationselektrode sowohl für die Oberflächenkoagulation, als auch die interstitielle Koagulation kann vorzugsweise die vorangehend beschriebene Temperiereinheit in allen Ihren Varianten beinhalten.

Die Erfindung wird nachfolgend anhand von Ausführungsbeispielen und den dazugehörigen Zeichnungen näher erläutert. Es zeigen:
- Figur 1: eine Draufsicht auf eine bipolare Koagulationssonde,
- Figur 2: einen Längsschnitt durch eine bipolare Koagulationssonde mit einer Temperiereinheit nach einer ersten Variante,
- Figur 3: einen Längsschnitt durch eine bipolare Koagulationssonde mit einer Temperiereinheit nach einer zweiten, nicht beanspruchten Variante mit partiellem Flüssigkeitsaustritt zur Verhinderung der Gewebsaustrocknung,
- Figur 4: ein Temperaturprofil während der Koagulation mit einer temperierten Koagulationssonde und
- Figur 5: einen Längsschnitt durch eine Koagulationssonde mit einer Temperiereinheit nach einer dritten Variante zur oberflächlichen und interstitiellen Koagulation.

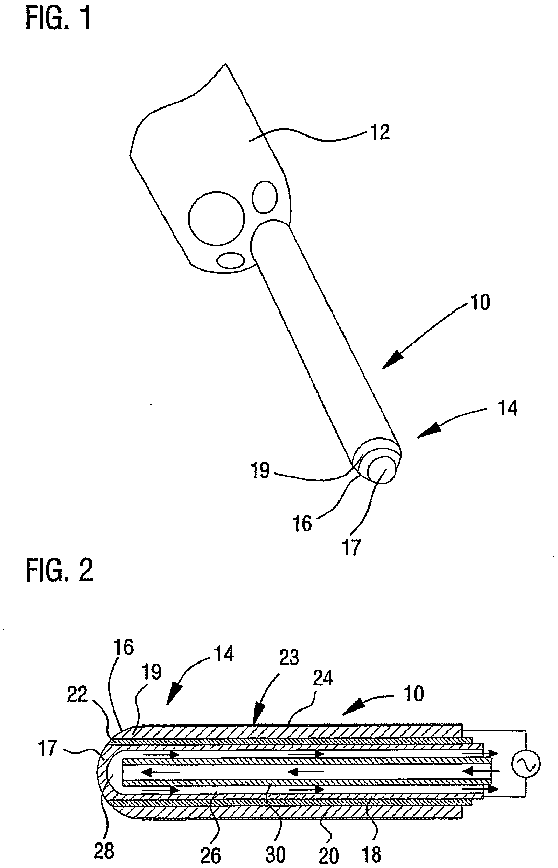

Die Figur 1 zeigt eine bipolare Koagulationssonde 10, die in einem Führungsschaft eines hier nicht näher erläuterten Endoskops 12 sitzt. Die Koagulationssonde 10 besitzt einen Schaft 23 mit einem distalen Ende 14, welches von einer sphärischen Stirnfläche 16 gebildet ist. In die sphärische Stirnfläche 16 sind zwei elektrisch voneinander isoliert, elektrische Pole 17, 19 einer ersten und zweiten Elektrode 18, 20 integriert. Die Koagulationssonde 10 kann zur Oberflächenkoagulation eingesetzt werden.

Figur 2 gibt schematisch in einem Längsschnitt den Aufbau der Koagulationssonde 10 aus Figur 1 wieder. Wie ersichtlich, ist die Stirnfläche 16 kugelförmig ausgebildet und gliedert sich an ihrer Oberfläche in Abschnitte, die von den Polen 17, 19 der ersten und zweiten Elektrode 18, 20 sowie einem die beiden Elektroden 18, 20 trennenden Isolator 22 gebildet sind. Die Stirnfläche 16 ist im Ausführungsbeispiel nahezu als Halbkugel ausgeformt, die einen abgerundeten Übergang zum Schaft 23 bildet.

Die erste Elektrode 18 liegt in Form einer an ihrem distalen Ende geschlossenen Metallhülse vor. Das geschlossene Ende der Metallhülse ist mittig in der Stirnfläche 16 der Elektrodenspitze 14 angeordnet und bildet dort den Pol 17. Die zweite Elektrode 20 hat die Form eines Metallrohres, das von einer dünnen Isolatorschicht 24 im Bereich des Schaftes 23 bedeckt ist. Ein distales Ende der zweiten Elektrode 20 bildet den Pol 19. Bei Anlegen einer Wechselspannung fließt nun im Bereich der sphärischen Stirnfläche 16 ein Strom über das anliegende Gewebe zwischen den beiden Polen 17, 19 der Elektroden 18, 20. Das Gewebe wird in Folge dessen erhitzt und koaguliert. Bei einer nicht temperierten Koagulationselektrode herrscht die höchste Temperatur in dem Gewebebereich, der an den Polen 17, 19 der Elektroden 18, 20 anliegt. Dies kann dazu führen, dass mit zunehmender Austrocknung ein allmähliches Anhaften des Gewebes an den Polen 17, 19 der Elektroden 18, 20 erfolgt.

Zur Vermeidung des vorgenannten Effektes ist in die Koagulationselektrode 10 eine Temperiereinheit 26 integriert. Die Temperiereinheit 26 umfasst eine an der Rückseite der Stirnfläche 16 angeordnete Leitstruktur 28, über die ein anströmendes Temperiermedium umgelenkt und zurückgeführt wird. Als Temperiermedium wird eine sterile, pyrogenfreie, physiologische Kochsalzlösung oder deionisertes Wasser eingesetzt. Die Temperiereinheit 26 umfasst vorliegend zur Realisation der Leitstruktur 28 einen etwa mittig im Schaft 23 angeordneten Schlauch 30, in dem das Temperiermedium bis zur Rückseite der Stirnfläche 16 von einer - hier nicht dargestellten - Schlauchpumpe gefördert wird, sowie eine an der Rückseite der Stirnfläche 16 angeordnete konkave Struktur. Die Leitstruktur 28 führt das Temperiermedium außen vorbei am Schlauch 30 zurück. Eine Fließrichtung des Temperiermediums ist durch die dargestellten Pfeile angedeutet. Bei der hier dargestellten Variante der Temperiereinheit 26 wird der Pol 19 der zweiten Elektrode 20 im Vergleich zum Pol 17 der ersten Elektrode 18 weniger effektiv gekühlt. In einem solchen Fall kann eine gewünschte Verringerung der Elektrodenoberflächentemperatur dadurch erreicht werden, dass die elektrischer Pol 19 der zweiten Elektrode 20 entsprechend größer gestaltet wird, um die Stromdichte und damit die Aufwärmung zu reduzieren. Die Temperiereinheit 26 umfasst ferner - hier ebenfalls nicht dargestellte - Mittel zum Temperieren des Temperiermediums. Diese Mittel können z.B. einen in den Kreislauf des Temperiermedium einschalteten Thermostaten gängiger Ausführung umfassen.

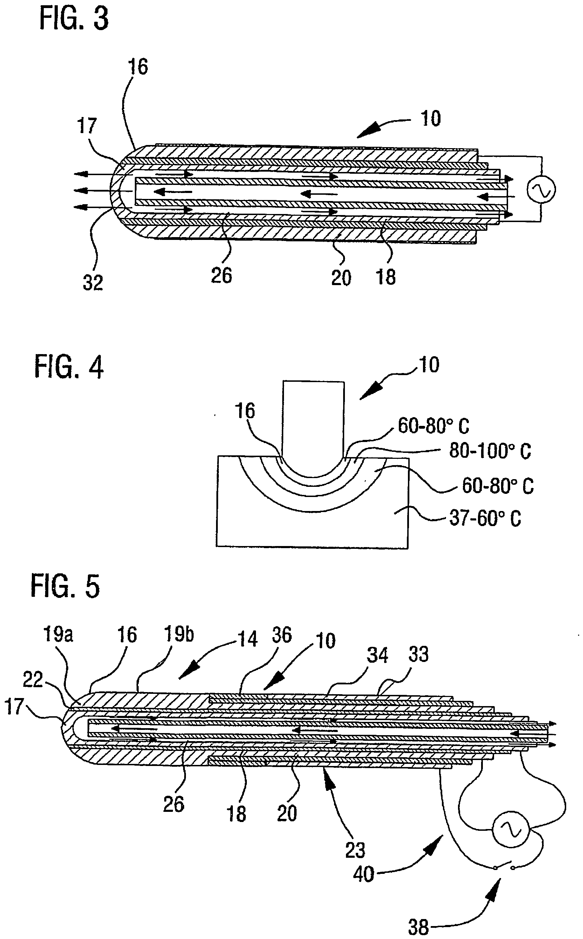

In der Figur 4 ist schematisch ein sich bei Temperierung der Koagulationselektrode 10 einstellendes Temperaturprofil dargestellt. Wie ersichtlich wird durch die Temperierung der - hier nicht detailliert dargestellten - Pole 17, 19 an der sphärischen Stirnflächen 16 eine für die Gewebeanhaftung kritische Temperatur nicht erreicht. Die Zone der kritischen Temperatur ist vielmehr in tiefere Gewebsschichten verschoben. Ein Ankleben des Gewebes kann auf diese Weise wirkungsvoll vermieden werden.

Die Figur 3 zeigt eine nicht beanspruchte Variante der Koagulationselektrode 10. Der Aufbau der Koagulationselektrode 10 entspricht im wesentlichen dem bereits in Figur 2 geschilderten Aufbau, sodass hierauf verwiesen wird. Einziger Unterschied besteht darin, dass der Bereich der sphärischen Stirnfläche 16 des Pols 17 der ersten Elektrode 18 kleine Bohrungen 32 aufweist, durch die das Temperiermedium der Temperiereinheit 26 austreten kann. Das Temperiermedium ist in diesem Falle eine sterile, pyrogenfreie, physiologische Kochsalzlösung. Ein Teil oder auch die gesamte Menge des durch die Koagulationselektrode 10 gepumpten Temperiermediums tritt aus den kleinen Bohrungen 32 in der Stirnfläche 16 aus und benetzt diesen Elektrodenbereich mit elektrisch leitender Flüssigkeit. Hierdurch kann die bei starker Erwärmung des Gewebes einsetzende Gewebsaustrocknung kompensiert und der elektrische Energieeintrag durch die Aufrecherhaltung der elektrischen Leitfähigkeit des Gewebes verbessert werden.

Die Koagulationselektrode 10 der Figur 5 vereint die Eigenschaften eines bipolar interstitiellen Koagulators und eines Oberflächenkoagulators und ist im Aufbau an die Variante der Figur 2 angelehnt. Der Pol 19 ist in dieser Ausführungsvariante in proximaler Richtung auf der Oberfläche des Schaftes verlängert, so dass sich neben der Polfläche 19a im Stirnbereich der Sonde eine weitere Polfläche 19b auf dem Umfang des Schaftes ergibt. Als Oberflächenkoagulator agieren die beiden an der Stirnfläche 16 angeordneten Pole 17, 19a der Elektroden 18, 20, während zur interstitiellen Koagulation eine Wechselspannung an die beiden Pole 19, 33 der Elektrode 20, 34 angelegt wird.

Die zweite Elektrode 20 weist einen Pol 19 mit einer Polfläche 19a sowohl im Bereich der sphärischen Stirnfläche 16, als auch einer Polfläche 19b im Umfang der Elektrodespitze 14, also am Schaft 23, auf. Für die interstitielle Koagulation wirken beide Polflächen 19a und 19b als ein Pol 19 zusammen. Die zweite Elektrode 20 ist durch einen Isolator 36 von der dritten Elektrode 34 getrennt. Die Temperiereinheit 26 entspricht der Temperiereinheit der Figur 2 und wird daher an dieser Stelle nicht näher erläutert.

Die in Figur 5 dargestellte kombinierte Koagulationssonde weist demnach insgesamt drei Elektroden 18, 20, 34 auf, die die Pole 17, 19a, 19b und 33 bilden. Durch Schließen des Schalters 38 der Schaltung 40 kann die Koagulationselektrode 10 von einem reinen Oberflächenkoagulator zu einem interstitiellen Koagulator umfunktioniert werden. Die Elektroden 18 und 34 liegen auf dem gleichen Potential, so dass sich mit Sicherheit eine Koagulation um die Elektrodenspitze 14 herum ausbildet. Bei der Wahl der Polgrößen ist zu berücksichtigen, dass die Elektrode 20 und insbesondere die Elektrode 34 nicht direkt temperiert werden, da sie nicht unmittelbar in Kontakt zum zirkulierenden Temperiermedium stehen, sondern durch mehrere Isolationsschichten 22, 36 sowie das Metallrohr der Elektrode 18 beziehungsweise das Metallrohr der Elektrode 20 vom Temperiermedium getrennt sind. Bei einer gewünschten Verringerung der Elektrodenoberflächentemperatur ist die Fläche der Pole 19a, 19b, 33 der Elektroden 20, 34 entsprechend größer zu gestalten, um die Stromdichte und somit die Aufwärmung zu reduzieren.

## Patentansprüche

1. Bipolare Koagulationssonde (10) mit einem proximalen und einem distalen Ende (14) und einer Stirnfläche (16) am distalen Ende (14) des Schaftes-, in die ein Pol (17) einer ersten Elektrode (18) und ein Pol (19) einer zweiten Elektrode (20) integriert sind, wobei die Koagulationssonde (10) eine Temperiereinheit (26) umfasst, die ausgebildet ist, die Koagulationssonde im Bereich ihres distalen Endes (14) mittels eines Temperiermediums zu temperieren,
- die zweite Elektrode (20) zumindest im /Bereich der Elektrodenspitze (14) rohrförmig ist, wobei ein distales Ende der rohrförmigen zweiten Elektrode (20) den Pol (19) in der Stirnfläche (16) ausbildet; und
- der Pol (19) der zweiten Elektrode (20) ringförmig ausgebildet ist und der Pol (17) der ersten Elektrode (18) innerhalb des ringförmigen Pols (19) der zweiten Elektrode angeordnet ist, **dadurch gekennzeichnet, dass** die erste Elektrode (18) in Form einer an ihrem distalen Ende geschlossenen Metallhülse vorliegt und das geschlossene Ende der Metallhülse mittig in der Stirnfläche (16) des distalen Endes (14) angeordnet ist und dor den Pol (17) ausbildet.

2. Koagulationssonde (10) nach Anspruch 1, **dadurch gekennzeichnet, dass** die Temperiereinheit (26) eine Leitstruktur (28) für das Temperiermedium umfasst, mit der das Temperiermedium zu einer Rückseite der Stirnfläche (16) zu führen und von dort umzulenken und zurückzuführen ist.

3. Koagulationssonde (10) nach Anspruch 2, **dadurch gekennzeichnet, dass** das Temperiermedium eine sterile, pyrogenfreie, physiologische Kochsalzlösung ist.

4. Koagulationssonde (10) nach Anspruch 2, **dadurch gekennzeichnet, dass** das Temperiermedium deionisiertes Wasser ist.

5. Koagulationssonde(10) nach Anspruch 1, **dadurch gekennzeichnet, dass** die Temperiereinheit (26) an eine Schlauchpumpe angeschlossen ist.

6. Koagulationssonde (10) nach einem der vorhergehenden Ansprüche, **dadurch gekennzeichnet, dass** die Koagulationselektrode (10) zumindest zwei Elektroden (20, 34) mit einem am Umfang der Elektrodenspitze (14) angeordneten Pol (19, 33) umfasst.

7. Koagulationssonde (10) nach Anspruch 6, **dadurch gekennzeichnet, dass** der am Umfang der Elektrodenspitze (14) angeordnete Pol (19, 33) der Elektrode (20, 34) ringförmig die Elektrodenspitze (14) umläuft.

8. Koagulationssonde (10) nach einem der Ansprüche 6 bis 7, **dadurch gekennzeichnet, dass** Elektrode (20) sowohl eine Polfläche (19a) in der Stirnfläche (16) als auch eine Polfläche (19b) am Umfang der Elektrodenspitze (14) aufweist.

9. Koagulationssonde (10) nach Anspruch 8 **dadurch gekennzeichnet, dass** die Koagulationselektrode (10) insgesamt drei Elektroden (18, 20, 34) enthält.

## Claims

1. Bipolar coagulation probe (10) having a proximal end and a distal end (14) and an end face (16) at the distal end (14) of the shank, in which end face (16) a pole (17) of a first electrode (18) and a pole (19) of a second electrode (20) are integrated, the coagulation probe (10) comprising a temperature-control unit (26) which is configured to temperature-control the coagulation probe in the region of its distal end (14) by means of a temperature-control medium,
- the second electrode (20) being tubular at least in the region of the electrode tip (14), a distal end of the tubular second electrode (20) forming the pole (19) in the end face (16) ; and
- the pole (19) of the second electrode (20) being in an annular form and the pole (17) of the first electrode (18) being arranged inside the annular pole (19) of the second electrode, **characterised in that** the first electrode (18) is in the form of a metal sleeve closed at its distal end and the closed end of the metal sleeve is arranged centrally in the end face (16) of the distal end (14) and there forms the pole (17).

2. Coagulation probe (10) according to claim 1, **characterised in that** the temperature-control unit (26) comprises a guide structure (28) for the temperature-control medium by means of which the temperature-control medium is to be guided to a rear side of the end face (16) and there deflected and guided back.

3. Coagulation probe (10) according to claim 2, **characterised in that** the temperature-control medium is a sterile, pyrogen-free, physiological saline solution.

4. Coagulation probe (10) according to claim 2, **characterised in that** the temperature-control medium is deionised water.

5. Coagulation probe (10) according to claim 1, **characterised in that** the temperature-control unit (26) is connected to a peristaltic pump.

6. Coagulation probe (10) according to any one of the preceding claims, **characterised in that** the coagulation electrode (10) comprises at least two electrodes (20, 34) having a pole (19, 33) arranged at the circumference of the electrode tip (14).

7. Coagulation probe (10) according to claim 6, **characterised in that** the pole (19, 33) of the electrode (20, 34) arranged at the circumference of the electrode tip (14) extends in an annular manner around the electrode tip (14) .

8. Coagulation probe (10) according to either claim 6 or claim 7, **characterised in that** electrode (20) has both a pole face (19a) in the end face (16) and a pole face (19b) at the circumference of the electrode tip (14).

9. Coagulation probe (10) according to claim 8, **characterised in that** the coagulation electrode (10) contains a total of three electrodes (18, 20, 34).

## Revendications

1. Sonde de coagulation bipolaire (10) présentant des extrémités proximale et distale (14) et une face frontale (16) à l'extrémité distale (14) de la tige, face dans laquelle sont intégrés un pôle (17) d'une première électrode (18) et un pôle (19) d'une seconde électrode (20), la sonde de coagulation (10) comportant une unité d'équilibrage en température (26) qui est conformée pour équilibrer en température la sonde de coagulation dans la région de son extrémité distale (14) au moyen d'un fluide d'équilibrage en température,
- la seconde électrode (20) étant de forme tubulaire au moins dans la région de la pointe d'électrode (14), une extrémité distale de la seconde électrode (20) formant le pôle (19) dans la face frontale (16) ; et
- le pôle (19) de la seconde électrode (20) ayant une conformation tubulaire et le pôle (17) de la première électrode (18) étant disposé à l'intérieur du pôle (19) de forme tubulaire de la seconde électrode,
**caractérisée en ce que** la première électrode (18) se présente sous la forme d'un manchon métallique fermé à son extrémité distale et **en ce que** l'extrémité fermée du manchon métallique est disposée au milieu dans la face frontale (10) de l'extrémité distale (14) et forme le pôle (17).

2. Sonde de coagulation (10) selon la revendication 1, **caractérisée en ce que** l'unité d'équilibrage en température (26) comporte une structure de guidage (28) qui est destinée au fluide d'équilibrage en température et avec laquelle le fluide d'équilibrage en température peut être amené à un côté arrière de la face frontal (16) puis dévié et ramené.

3. Sonde de coagulation (10) selon la revendication 2, **caractérisée en ce que** le fluide d'équilibrage en température est de l'eau salée stérile, physiologique et dépourvue de pyrogène.

4. Sonde de coagulation (10) selon la revendication 2, **caractérisée en ce que** le fluide d'équilibrage en température est de l'eau déionisée.

5. Sonde de coagulation (10) selon la revendication 1, **caractérisée en ce que** l'unité d'équilibrage en température (26) est raccordée à une pompe tubulaire.

6. Sonde de coagulation (10) selon l'une des revendications précédentes, **caractérisée en ce que** l'électrode de coagulation (10) comporte au moins deux électrodes (20, 34) dont un pôle (19, 33) est disposé à la périphérie de la pointe d'électrode (14).

7. Sonde de coagulation (10) selon la revendication 6, **caractérisée en ce que** le pôle (19, 33) de l'électrode (20, 34) qui est disposé à la périphérie de la pointe d'électrode (14) s'étend annulairement autour de la pointe d'électrode (14).

8. Sonde de coagulation (10) selon l'une des revendications 6 à 7, caractérisée en qu'une électrode (20) comporte une face polaire (19a) dans la face frontale ainsi qu'une face polaire (19b) à la périphérie de la pointe d'électrode (14).

9. Sonde de coagulation (10) selon la revendication 8, **caractérisée en ce que** l'électrode de coagulation (10) contient en tout trois électrodes (18, 20, 34).
